(19) Europäisches Patentamt
European Patent Office
Office européen des brevets

(11) Veröffentlichungsnummer : **0 429 751 B1**

(12) **EUROPÄISCHE PATENTSCHRIFT**

(45) Veröffentlichungstag der Patentschrift :
**16.06.93 Patentblatt 93/24**

(51) Int. Cl.$^5$ : **C07D 417/04,** A61K 31/54,
// C07D271/04, A61K31/41

(21) Anmeldenummer : **90112002.2**

(22) Anmeldetag : **25.06.90**

(54) **Substituierte 3-Aminosydnonimine, Verfahren zu ihrer Herstellung und ihre Verwendung.**

(30) Priorität : **30.11.89 DE 3939515**
**30.11.89 DE 3939550**

(43) Veröffentlichungstag der Anmeldung :
**05.06.91 Patentblatt 91/23**

(45) Bekanntmachung des Hinweises auf die Patenterteilung :
**16.06.93 Patentblatt 93/24**

(84) Benannte Vertragsstaaten :
**AT BE CH DE DK ES FR GB IT LI NL SE**

(56) Entgegenhaltungen :
**EP-A- 0 023 343**
**EP-A- 0 312 773**
**EP-A- 0 327 808**
**EP-A- 0 346 694**

(73) Patentinhaber : **CASSELLA Aktiengesellschaft**
**Hanauer Landstrasse 526**
**W-6000 Frankfurt am Main 61 (DE)**

(72) Erfinder : **Schönafinger, Karl, Dr.**
**Holunderweg 8**
**W-8755 Alzenau (DE)**
Erfinder : **Beyerle, Rudi, Dr.**
**Mitterfeld 5d**
**W-8132 Tutzing (DE)**
Erfinder : **Bohn, Helmut, Dr.**
**Kranzbergring 11**
**W-6369 Schöneck (DE)**
Erfinder : **Just, Melitta, Dr.**
**Theodor-Heuss-Strasse 80**
**W-6070 Langen (DE)**

(74) Vertreter : **Urbach, Hans-Georg, Dr. et al**
**CASSELLA AKTIENGESELLSCHAFT,**
**Patentabteilung, Hanauer Landstrasse 526**
**W-6000 Frankfurt am Main 60 (DE)**

EP 0 429 751 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft pharmakologisch wirksame substituierte 3-Aminosydnonimine der allgemeinen Formel I

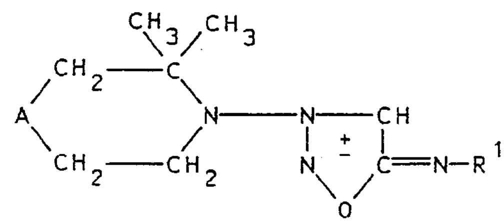

( I )

und ihre pharmakologisch annehmbaren Säureadditionssalze, worin
A den Rest -S- oder $-SO_2-$,
$R^1$ den Rest $-CO-R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten.

Die Erfindung betrifft ferner Verfahren zur Herstellung der erfindungsgemäßen Verbindungen und ihre Verwendung.

Alkylreste, Alkoxyalkylreste und Alkoxyreste können geradkettig oder verzweigt sein. Dies gilt auch, wenn sie als Substituenten für Phenyl auftreten.

($C_1$ bis $C_4$)Alkylreste können sein: Methyl, Ethyl, n-Propyl, i-Propyl, n-Butyl, i-Butyl, tert.-Butyl, sec.-Butyl. ($C_1$ bis $C_4$)Alkoxyreste können z.B. sein: Methoxy, Ethoxy, n-Propoxy, i-Propoxy, n-Butoxy, i-Butoxy, sec.-Butoxy, tert.-Butoxy.

($C_1$ bis $C_4$)Alkoxy-($C_1$ bis $C_4$)alkylreste können z.B sein: Methoxy-methyl, Ethoxy-methyl, Propoxy-methyl, Butoxy-methyl, i-Butoxy-methyl, 2-Methoxy-ethyl, 3-Methoxy-propyl, 4-Methoxy-butyl, 2-Ethoxy-ethyl, 3-Propoxy-propyl, 4-Propoxy-butyl, 2-Butoxy-ethyl, 3-Butoxy-propyl, 4-Butoxy-butyl, 3-i-Propoxy-propyl, 4-i-Propoxy-butyl, 2-Ethoxy-ethyl, 2-Propoxy-ethyl, 2-i-Propoxy-ethyl, 2-Butoxy-ethyl, 2-i-Butoxy-ethyl, 1-Methoxy-ethyl, 1-Ethoxy-ethyl, 1-Propoxy-ethyl, 2-Methoxy-propyl, 2-Ethoxy-propyl, 2-Propoxy-propyl, 2-i-Propoxy-propyl, 3-Methoxy-butyl, 3-Ethoxy-butyl, 3-Propoxy-butyl, 3-i-Propoxy-butyl, 3-Butoxy-butyl, 3-i-Butoxy-butyl, 3-tert.-Butoxy-butyl, 2-Methoxy-butyl, 2-Ethoxy-butyl, 2-Propoxy-butyl, 2-i-Propoxy-butyl, 2-Butoxy-butyl, 2-i-Butoxy-butyl.

Von den ($C_5$ bis $C_7$)Cycloalkylresten sind Cyclopentyl und Cyclohexyl bevorzugt.

Als Halogenatome für den substituierten Phenylrest kommen Fluor, Chlor, Brom und/oder Iod in Betracht, von denen Brom und Chlor bevorzugt sind.

$R^2$ bedeutet vorzugsweise ($C_1$ bis $C_4$)Alkyl, ($C_1$ bis $C_4$)-Alkoxy, Phenyl, oder durch Chlor, Methyl oder Methoxy monosubstituiertes Phenyl, wobei das monosubstituierte Phenyl vorzugsweise in 4- oder 2- Stellung substituiert ist.

Für $R^1$ ist Wasserstoff bevorzugt. Bevorzugte Verbindungen sind: N-p-Anisoyl-3-(3,3-dimethyl-tetrahydro-1,4-thiazin-4-yl)-sydnonimin, sowie die pharmakologisch annehmbaren Säureadditionssalze der vorgenannten Verbindung, insbesondere ihr Hydrochlorid, sowie die pharmakologisch annehmbaren Säureadditionssalze, insbesondere die Hydrochloride der Verbindungen: 3-(3,3-Dimethyl-1,4-tetrahydrothiazin-1,1-dioxid-4-yl)-sydnonimin und 3-(3,3-Dimethyl-tetrahydro-1,4-thiazin-4-yl)-sydnonimin.

Eine Verbindung der allgemeinen Formel I kann dadurch hergestellt werden, daß eine Verbindung der allgemeinen Formel II

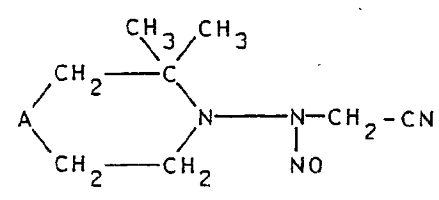

( II )

worin A die bereits genannte Bedeutung besitzt, zu einer Verbindung der allgemeinen Formel Ia

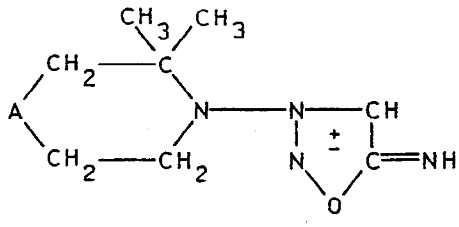

(Ia)

cyclisiert und die erhaltene Verbindung als Säure-additionssalz isoliert wird, oder daß für den Fall, daß eine Verbindung der Formel I mit $R^1$ = -$COR^2$ hergestellt werden soll, die Verbindung Ia oder ein Säureadditionssalz davon mit einem Acylierungsmittel, das den Rest -$COR^2$ einführt, acyliert wird und die so erhaltene Verbindung isoliert oder gegebenenfalls in ein pharmakologisch annehmbares Säureadditionssalz überführt, und dieses isoliert wird.

Die Cyclisierung der Verbindungen II zu den Verbindungen Ia kann in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt werden.

Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt.

Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methylethylether, Di-n-propyl-ether, Di-isopropyl-ether, Methyl-n-butylether, Methyl-tert-butylether, Ethyl-propyl-ether, Di-butylether, Tetrahydrofuran, 1,4-Dioxan, 1,2-Dimethoxyethan, Bis-β-methoxyethylether; Oligoethylenglykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl-oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butylester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig- und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Acylierung der Verbindung der Formel Ia, die auch in Form eines Säureadditionssalzes vorliegen kann, zur Einführung des Restes $R^1$ = -$COR^2$ kann in an sich bekannter Weise mit einem geeigneten Acylierungsmittel der Formel III durchgeführt werden

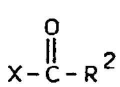

(III)

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl-oder Nitrophenyl-Rest ist; -O-CO-$R^2$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasiaromatischen Fünfring.

Die Acylierung wird zweckmäßigerweise in flüssiger oder flüssiger disperser Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III theoretisch 1 : 1. Das Acylierungsmittel kann jedoch auch im Über- oder Unterschuß eingesetzt werden. Zweckmäßigerweise wird das Acylierungsmittel der Formel III im Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkalimetallhydroxids, wie z.B. Natrium-, Kalium- oder Lithiumhydroxid, eines tertiären organischen Amins, wie z.B. Pyridin oder Triethylamin, eines Alkalimetallcarbonats oder Alkalimetallbicarbonats, wie z.B. Soda oder Natriumbicarbonat, oder eines Alkalimetallsalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen: Säurehalogenide bzw. Halogenameisensäureester, von denen Säurechloride und Chlorameisensäureester bevorzugt sind; für -OH: Carbonsäuren; für -O-Alkyl und -O-Aryl: Ester, von denen die Tolyl-, 2,4-Dinitro- oder 4-Nitrophenylester bevorzugt sind; für -O-CO-R: Anhydride; für -O-CO-O-Alkyl; gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol(1,2,4), N,N'-Carbonyl-dipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyl- oder N-Methyl-N'-tert.Butylcarbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N'-Disuccinimidyl-carbonat, Diphthalimidyl-carbonat, 1,1'-(Carbonyldioxy)-dibenzotriazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonyl-benzimidazol oder N,N'-Carbonylbenztriazol, geeignet (vgl. z.B. H.A. Staab, M. Lücking und F.H. Dürr, loc. Cit; H.A. Staab und A. Mannschreck loc. Cit.; H.A. Staab und W. Rohr loc. Cit). Als N,N'-Carbonyl-diazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-Amino-sydnonimine der allgemeinen Formel I können mit anorganischen oder organischen Säuren Säureadditionssalze bilden. Zur Bildung derartiger Säureadditionssalze sind anorganische oder organische Säuren geeignet. Für die Bildung pharmakologisch annehmbarer Säureadditionssalze sind geeignete Säuren beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der Formel Ia sind in freier Form nicht beständig, sondern werden in Form ihrer Säureadditionssalze isoliert. Bei der Synthese der Verbindungen der Formel Ia fallen normalerweise die Säureadditionssalze an.

Die benötigten Ausgangsverbindungen der allgemeinen Formel II können in an sich bekannter Weise nach der Strecker'schen Aminonitrilsynthese aus Verbindungen der allgemeinen Formel IV oder ihren Säureadditionssalzen

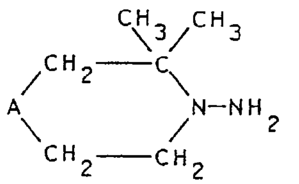

(IV)

worin A die bereits genannte Bedeutung besitzt, durch Umsetzung mit Formaldehyd und Blausäure bzw. Natriumcyanid in einem geeigneten Lösungs- oder Dispergiermittel oder Lösungs- oder Dispergiermittelgemisch, z.B. in Wasser, hergestellt werden, wobei zunächst eine Verbindung der allgemeinen Formel V

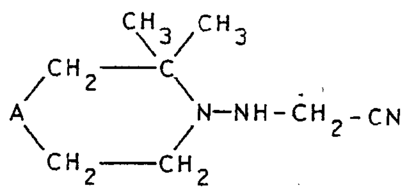

(V)

entsteht, die durch Nitrosierung in die Verbindung II überführt wird. Die Nitrosierung wird in bekannter Weise in einem geeigneten Lösungs- oder Dispergiermittel oder Lösungs- oder Dispergiermittelgemisch, z.B. in Wasser, z.B. bei Temperaturen von -10 bis 10°C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen. Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV lassen sich, ausgehend von Verbindungen der allgemeinen Formel VI

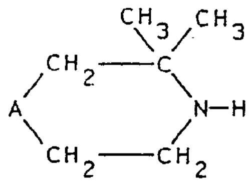

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird:

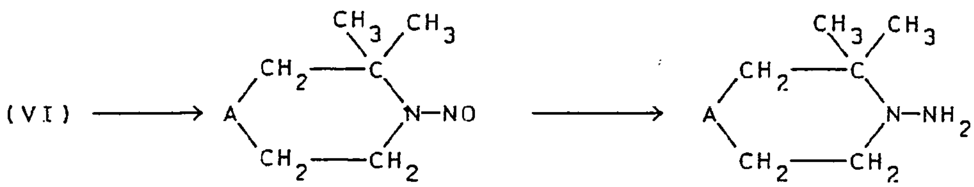

(VII) (IV)

oder daß in an sich bekannter Weise

b) eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird:

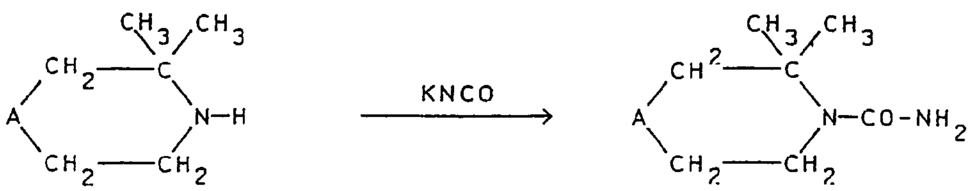


(VI) (VIII)

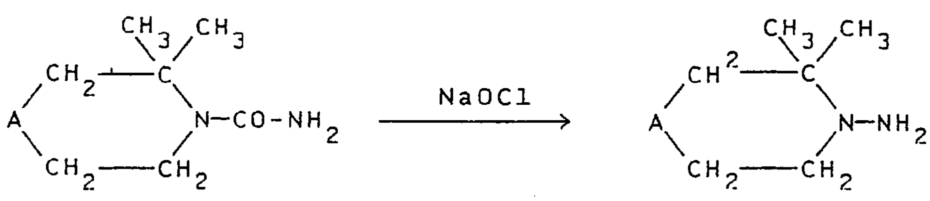


(VIII) (IV).

Die Verbindung 3,3-Dimethyl-tetrahydro-1,4-thiazin (A = -S-in Formel VI) kann durch Umsetzung von 2,2-Dimethylaziridin (herstellbar durch Wasserabspaltung aus 2-Amino-2-methyl-propan-1-ol) mit 3-Mercaptoethanol hergestellt werden, wobei sich zunächst (2-Hydroxy-ethyl)-(2-amino-2-methyl-propyl)-sulfid bildet. An dieser Verbindung wird die Hydroxylgruppe durch Chlorierung, z.B. mit Thionylchlorid, durch Chlor ersetzt und die so erhaltene Verbindung anschließend durch Einwirkung einer Base cyclisiert.

Die Verbindung 4-Amino-3,3-dimethyl-1,4-tetrahydrothiazin-1,1-dioxid (A = $-SO_2-$ in Formel IV) ist bekannt (vgl. Arzneimittelforschung 22, (1972), 1568). Sie läßt sich wie folgt herstellen: Monothioethylenglycol und 2,2-Dimethyloxiran werden in Gegenwart von katalytischen Mengen Alkalihydroxid bei erhöhter Temperatur zu (2-Hydroxy-2-methylpropyl)-(2-hydroxy-ethyl)-sulfid umgesetzt, dieses mit $H_2O_2$ in das entsprechende (2-Hydroxy-2-methylpropyl)-(3-hydroxy-ethyl)-sulfon umgewandelt und dieses in Gegenwart eines wasserentziehenden Mittels, wie z.B. Kaliumhydrogensulfat zum 2,2-Dimethyl-1,4-oxathian-4,4-dioxid der Formel IX cyclisiert:

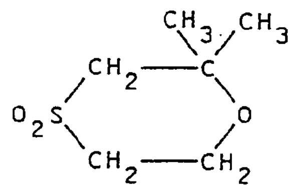

(IX)

Diese Verbindung läßt sich mit Hydrazinhydrat zu der Verbindung IV mit A = $-SO_2-$ umsetzen, vgl. Arzneimittelforschung loc. Cit. und nachfolgendes Beispiel 1a.

Erfindungsgemäße Verbindungen der Formel I mit A = $-SO_2-$können auch dadurch hergestellt werden, daß eine erfindungsgemäße Verbindung der Formel I mit A = -S- oder eine Verbindung der Formel I mit A = -SO-, oder ein Säureadditionssalz einer solchen Verbindung, mit einer Sauerstoff abgebenden Substanz umgesetzt und die erhaltene Verbindung gegebenenfalls in ein Säureadditionssalz überführt und isoliert wird, oder daß für den Fall, daß eine Verbindung der Formel I mit A = $-SO_2-$ und $R^1$ = H erhalten worden ist und eine Verbindung der Formel I mit A = $-SO_2-$ und $R^2$ = $-COR^2$ hergestellt werden soll, die erhaltene Verbindung oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt und dieses isoliert wird. Als geeignete Sauerstoff abgebende Substanzen sind beispielsweise zu nennen: Wasserstoffperoxid, Peroxosäuren, wie z.B. Perameisen-, Peressig-, Perbenzoe-, m-Chlorperbenzoe-, Perphthal-säure, sowie Diacylperoxide. Auch Gemische verschiedener Sauerstoff abgebender Substanzen können verwendet werden. Die Sauerstoff abgebende Substanz wird mindestens in der stöchiometrisch erforderlichen Menge eingesetzt. Die Umsetzung kann zweckmäßigerweise in einem geeigneten Lösungs- oder Dispergiermittel oder Lösungs-oder Dispermittelgemisch bei Temperaturen von 0°C, vorzugsweise von Raumtemperatur, bis zum Siedepunkt des Lösungs- oder Dispergiermittels oder Lösungs- oder Dispergiermittelgemischs, durchgeführt werden. Geeignete Lösungs- oder Dispergiermittel sind z.B. die bereits genannten Ether, aliphatische, cycloaliphatische und aromatische Kohlenwasserstoffe, halogenierte aliphatische und aromatische Kohlenwasserstoffe, Wasser, insbesondere aber niedere Carbonsäuren, wie Ameisen-oder Essigsäure.

Eine nach der Umsetzumg mit dem Sauerstoff abgebenden Mittel gewünschtenfalls durchzuführende Acylierung mit einem den Rest $-COR^2$ einführenden Acylierungsmittel wird in der bereits genannten Art und Weise mit einem Acylierungsmittel der Formel III durchgeführt.

Die gewünschtenfalls vorzunehmende Überführung in das Säureadditionssalz wird, wie bereits vorstehend angegeben, in an sich bekannter Weise durchgeführt.

Die Verbindungen der Formel I mit A = $-SO_2-$ können analog den Verbindungen der Formel I mit A = -S- aus Verbindungen der Formel II mit A = $-SO_2-$ hergestellt werden.

Die erfindungsgemäßen Verbindungen der allgemeinen Formel I und ihre pharmakologisch annehmbaren Säureadditionssalze besitzen wertvolle pharmakologische z.B. hämodynamische, thrombozytenfunktionshemmende und antithrombotische, insbesondere coronar-antithrombotische Eigenschaften. Besonders ausgeprägt ist ihre Wirkung auf das Herz-Kreislauf-System. Verglichen mit bekannten, in 3-Stellung strukturähnlichen substituierten Sydnoniminverbindungen, z.B. solchen der FR-PS-1496056 und den Verbindungen der älteren deutschen Patentanmeldung P 3820210.7 übereinstimmend mit der EP-A-0346 694, welche unter Artikel 54(3) EPÜ fällt, besitzen sie überraschenderweise eine erstaunlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die erfindungsgemäßen Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer erfindungsgemäßen Verbindung der Formel I oder eines Säureadditionssalzes davon, neben einem oder mehreren üblichen pharmazeutisch einwandfreien Träger-oder Verdünnungs- und gegebenenfalls einem oder mehreren Zusatzstoffen enthalten.

Die Heilmittel können oral, z.B. in Form von Tabletten, Filmtabletten, Dragees, Hart- und Weichgelatinekapseln, Mikrokapseln, Granulaten, Pulvern, Pellets, Lösungen, Sirupen, Emulsionen, Suspensionen, Aerosolen, Schäumen, Pillen oder Pastillen verabreicht werden. Die Verabreichung kann aber auch rektal, z.B. in Form von Suppositorien, oder parenteral, z.B. in Form von Injektionslösungen, oder perkutan, z.B. in Form von Salben, Cremes, Gelen Pasten, Aerosolen, Schäumen, Pudern, Tinkturen, Linimenten oder sog. Transdermalen Therapeutischen Systemen (TTS) erfolgen.

Die pharmazeutischen Präparate können in an sich bekannter Weise unter Verwendung pharmazeutisch

inerter anorganischer oder organischer Hilfs-, Träger-, Füll- oder Verdünnungsstoffe hergestellt werden. Für die Herstellung von Pillen, Tabletten, Filmtabletten, Dragees und die Pellet- oder Granulatfüllungen von Hartgelatinekapseln kann man z.B. Calciumphosphate, Lactose, Sorbitol, Mannitol, Stärken, präparierte Stärken, chemisch modifizierte Stärken, Stärkehydrolysate, Cellulose, Cellulosederivate, synthetische Polymere, Talk etc. verwenden. Träger- oder Verdünnungsstoffe für Weichgelatinekapseln und Suppositorien sind z.B. Fette, Wachse, halbfeste und flüssige Polyole, natürliche oder gehärtete Öle etc. Als Träger- oder Verdünnungsstoffe für die Herstellung von Lösungen und Sirupen eignen sich z.B. Wasser, Polyole, Lösungen von Saccharose, Invertzucker, Glukose etc. Als Trägerstoffe für die Herstellung von Injektionslösungen eignen sich z.B. Wasser, Alkohole, Glyzerol, Polyole oder pflanzliche Öle. Als Träger- oder Verdünnungsstoffe für Salben, Cremes und Pasten eignen sich z.B. Naturvaseline, Kunstvaseline, dick- und dünnflüssige Paraffine, Fette, natürliche oder gehärtete pflanzliche und tierische Öle, Neutralöle, Wachse, Wachsalkohole, Polyethylenglykole, Polyacrylsäure, Silicongele etc.

Die pharmazeutischen Präparate können in an sich bekannter Weise neben den Wirk- und Verdünnungs-, Füll- oder Trägerstoffen auch noch einen oder mehrere Zusatzstoffe oder Hilfsmittel, wie z.B. Spreng-, Binde-, Gleit-, Schmier-, Formtrenn-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler, Lösungsbeschleuniger, Antischaummittel, Salzbildner, Gelbildner, Verdickungsmittel, Fließregulierungsmittel, Sorptionsmittel, Mittel zur Erzielung eines Depoteffekts oder Mittel, insbesondere Salze, zur Veränderung des osmotischen Drucks, Überzugsmittel oder Antioxidantien etc. enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen können beispielsweise sein: β-Rezeptorenblocker, wie z.B. Propranolol, Pindolal, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

In den pharmazeutischen Präparaten kann der Gehalt an dem Wirkstoff oder den Wirkstoffen der Formel I in weiten Grenzen schwanken und z.B. 0,05 bis 50 Gew.%, vorzugsweise 0,05 bis 20 Gew.%, betragen. In festen Darreichungsformen, wie Dragees, Tabletten etc. beträgt der Gehalt an einem oder mehreren Wirkstoffen der Formel I in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis 1 Gew.%, eines oder mehrerer Wirkstoffe der Formel I. Der Gehalt an einem oder mehreren Wirkstoffen der Formel I kann gegebenenfalls in den pharmazeutischen Präparaten teilweise, z.B. bis zu 50 Gew.%, vorzugsweise zu 5 bis 40 Gew.%, durch eine oder mehrere andere therapeutisch wirksame Substanzen ersetzt sein.

Die erfindungsgemäßen Verbindungen der Formel I, ihre pharmakologisch annehmbaren Säureadditionsssalze und pharmazeutische Präparate, welche die erfindungsgemäßen Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks; bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg-/Mensch. Die Tagesdosis wird normalerweise in mehrere, z.B. 2 bis 4 Teilverabreichungen aufgeteilt.

Die pharmakologische Wirkung der erfindungsgemäßen Verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). Die Wirkungsdauer der Prüfsubstanz ergibt sich aus der Zeit, die nach der Zugabe der Prüfsubstanz benötigt wird, bis der Ausgangswert wieder erreicht ist. In der folgenden Tabelle sind die so erhaltenen Werte angegeben.

$IC_{50}$-Werte und Wirkungsdauer für Sydnonimine der Formel

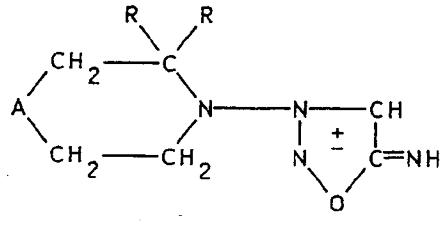 · HCl

| Verbindung Nr. | A | R | $IC_{50}$ in mol/l | Wirkungsdauer in min |
|---|---|---|---|---|
| 1 | 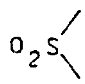 | $CH_3$ | $6 \cdot 10^{-6}$ | 240 |
| 2 | 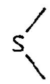 | $CH_3$ | $2 . 10^{-6}$ | 200 |
| 3 | 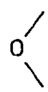 | H | $1 \cdot 10^{-6}$ | 80 |
| 4 | 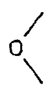 | $CH_3$ | $1 \cdot 10^{-6}$ | 120 |

Bei der Verbindung Nr. 1 der Tabelle handelt es sich um die bevorzugte erfindungsgemäße Verbindung 3-(3,3-Dimethyl-1,4-tetrahydrothiazin-1,1-dioxid-4-yl)-sydnonimin-hydrochlorid.

Bei der Verbindung Nr. 2 der Tabelle handelt es sich um die bevorzugte Verbindung aus Beispiel 5.

Bei der Verbindung Nr. 3 der Tabelle handelt es sich um die aus Beispiel 2 der FR-PS-1406056 bekannte Verbindung 3-Morpholino-sydnonimin-hydrochlorid.

Bei der Verbindung Nr. 4 der Tabelle handelt es sich um die Verbindung 3-(3,3-Dimethylmorpholin-4-yl)-sydnonimin-hydrochlorid des Beispiels Nr. 1 der älteren deutschen Patentanmeldung P 3820210.7.

Wie aus der vorstehenden Tabelle zu ersehen ist, besitzen die erfindungsgemäßen Verbindungen bei vergleichbarem $IC_{50}$-Wert eine wesentlich längere Wirkungsdauer als die beiden Vergleichsverbindungen.

Bestimmt man die Wirkungsdauer der Verbindungen bei der Konzentration von $6 \cdot 10^{-6}$ mol/l, so erhält man die nachstehend angegebenen Werte:

| Verbindung Nr. | Wirkungsdauer in min. |
|---|---|
| 1 | 240 |
| 2 | 220 |
| 3 | 100 |
| 4 | 140 |

Aus diesen Werten ist zu ersehen, daß die erfindungsgemäßen Verbindungen auch bei gleicher Dosis eine erheblich längere Wirkungsdauer besitzen als die Vergleichsverbindungen.

Soweit in den nachfolgenden Beispielen nichts anderes angegeben ist, handelt es sich bei Prozentangaben um Gewichtsprozente.

Beispiel 1

3-(3,3-Dimethyl-1,4-tetrahydrothiazin-1,1-dioxid-4-yl)-sydnonimin-hydrochlorid

a) 4-Amino-3,3-dimethyl-1,4-tetrahydrothiazin-1,1-dioxid-hydrogensulfat

Der Mischung aus 57 g 2,2-Dimethyl-1,4-oxathian-4,4-dioxid der Formel IX

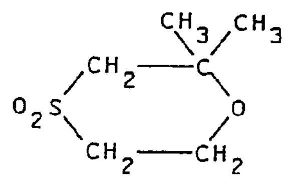

(IX)

45,5 g Hydrazinhydrat und 650 ml Wasser wird 6 h im Autoklaven auf 180°C erhitzt. Nach dem Erkalten wird die Mischung am Rotationsverdampfer eingeengt und der Rückstand in möglichst wenig Wasser gelöst, mit 50 %iger Schwefelsäure sauer gestellt und bei 0°C gerührt. Der Niederschlag wird abgesaugt, mit Ethanol gewaschen und getrocknet.
Ausbeute: 25 g Fp. 206°C (Zers.)

b) 4-Cyanmethylamino-3,3-dimethyl-1,4-tetrahydrothiazin-1,1-dioxid

Die Lösung von 26 g 4-Amino-3,3-dimethyl-1,4-tetrahydrothiazin-1,1-dioxid-hydrogensulfat (Stufe a) in 300 ml Wasser wird auf 5°C abgekühlt und mit 7,8 g Kaliumcyanid versetzt. Durch Zugabe von Sodalösung wird ein pH von 7 bis 7,5 eingestellt und 7,7 g einer 39 %igen Formalinlösung zugefügt. Die Mischung wird dann bei Raumtemperatur 6 h gerührt, wobei der pH durch Zugabe von etwas Salzsäure oder Sodalösung bei 7 gehalten wird. Das Produkt wird durch zweimaliges Ausschütteln mit Essigsäure-ethylester abgetrennt und bleibt nach dem Trocknen und Einengen des Lösungsmittels als Öl zurück, das nach kurzer Zeit erstarrt.
Ausbeute: 15 g Fp. 84 bis 85°C

c) 3-(3,3-Dimethyl-1,4-tetrahydrothiazin-1,1-dioxid-4-yl)-sydnonimin-hydrochlorid

Zur Mischung, bestehend aus 14 g 4-Cyanmethylamino-3,3-dimethyl-1,4-tetrahydrothiazin-1,1-dioxid (Stufe b), 200 ml Eiswasser, 10 ml 10 n Salzsäure und 200 ml Essigsäureethylester werden unter Kühlung und Stickstoff 6,8 g Natriumnitrit gegeben und bei Raumtemperatur 1 h weitergerührt. Die organische Phase wird abgetrennt, getrocknet und bei 0°C mit 30 ml 25 %iger isopropanolischer Salzsäure versetzt. Nach 15 h wird im Wasserstrahlvakuum einge engt und mit 500 ml Essigsäure-ethylester aufgekocht. Nach dem Erkalten wird abgesaugt und der Feststoff getrocknet.
Ausbeute: 5,5g Fp. 220°C Zers.
Analyse: $C_8H_{15}ClN_4O_3S$

| | C | H | N | O |
|---|---|---|---|---|
| Ber. | 34,0 | 5,3 | 19,8 | 17,0 |
| Gef. | 33,7 | 5,4 | 20,0 | 16,7 |

Die in der Stufe a benötigte Ausgangsverbindung IX kann wie folgt hergestellt werden (vgl. Arzneimittelforschung loc. Cit.):

In 1 mol Ethanol wird 1 mol KOH gelöst und 1 mol 2-Mercaptoethanol zugegeben. Zu dieser Lösung wird innerhalb von 60 min und unter Kühlung 1 mol 2,2-Dimethyloxiran zugetropft. Dann wird neutralisiert, abfiltriert, eingeengt und im Vakuum destilliert. Man erhält (2-Hydroxy-2-methyl-propyl)-(2-hydroxyethyl)-sulfid vom Siedepunkt 93°C bei 0,199 mbar.

Zu dem erhaltenen Sulfid werden 0,5 Gew.% Phosphorsäure zugegeben und danach das Sulfid durch Zutropfen von 30 gew.%igem $H_2O_2$ zum (2-Hydroxy-2-methyl-propyl)-(2-hydroxyethyl)-sulfon oxidiert. Man erhält dieses Sulfon nach dem Einengen als nichtdestilierbares Öl.

Ein Gemisch aus 32 g (2-Hydroxy-2-methyl-propyl)(2-hydroxy-ethyl)-sulfon und 5 g $KHSO_4$ werden auf 120°C erhitzt, nach Beendigung der Cyclisierung wird abgekühlt, abfiltriert, mit Pottasche neutralisiert, wieder abfiltriert, eingeengt und im Vakuum bei 0,532 mbar destilliert. Die Verbindung 2,2-Dimethyl-1,4-oxathian-4,4-dioxid hat bei diesem Druck einen Siedepunkt von
100 - 102°C. Fp: 83°C

Beispiel 2

N-Ethoxycarbonyl-3-(3,3-dimethyl-1,4-tetrahydro-thiazin-1,1-dioxid-4-yl)-sydnonimin

Die auf 0 bis 5°C abgekühlte Lösung von 1,5 g 3-(3,3-Dimethyl-1,4-tetrahydro-thiazin-1,1-dioxid-4-yl)-sydnonimin-hydrochlorid (Beispiel 1) in 20 ml Wasser wird mit 1,11 g $NaHCO_3$ und der Lösung von 0,65 g Chlorameisensäureethylester in 20 ml Methylenchlorid versetzt und bis zum Ende der Gasentwicklung bei bis auf Raumtemperatur ansteigender Temperatur gerührt. Die organische Phase wird abgetrennt, getrocknet und im Wasserstrahlvakuum eingeengt, der Rückstand aus Essigsäure-ethylester um-kristallisiert.
Ausbeute: 0,82 g Fp. 188-190°C
Analyse: $C_{11}H_{18}N_4O_5S$

| | C | H | N | O |
|---|---|---|---|---|
| Ber. | 41,5 | 5,7 | 17,6 | 25,2 |
| Gef. | 41,5 | 5,6 | 17,8 | 24,9 |

Beispiel 3

N-p-Anisoyl-3-(3,3-dimethyl-1,4-tetrahydro-thiazin-1,1-dioxid-4-yl)-sydnonimin

Die Verbindung wird analog Beispiel 2 mit p-Anisoylchlorid anstelle von Chlorameisensäurethylester erhalten.
Fp.: 178 bis 180°C
Analyse: $C_{16}H_{20}N_4O_5S$

| | C | H | N | O |
|---|---|---|---|---|
| Ber. | 50,5 | 5,3 | 14,7 | 21,1 |
| Gef. | 50,2 | 5,4 | 15,0 | 20,8 |

Beispiel 4

N-Cyclohexylcarbonyl-3-(3,3-dimethyl-1,4-tetrahydro-thiazin-1,1-dioxid-4-yl)-sydnonimin

Die Verbindung wird analog Beispiel 2 mit Cyclohexancarbonsäurechlorid anstelle von Chlorameisensäurethylester erhalten.
Fp.: 150 bis 151°C
Analyse: $C_{15}H_{24}N_4O_4S$

| | C | H | N | O |
|---|---|---|---|---|
| Ber. | 50,6 | 6,7 | 15,7 | 18,0 |
| Gef. | 50,5 | 6,5 | 15,8 | 18,0 |

Beispiel 5

3-(3,3-Dimethyl-tetrahydro-1,4-thiazin-4-yl)-sydnonimin-hydrochlorid

a) 2,2-Dimethylaziridin

100 g 2-Amino-2-methyl-propan-1-ol werden in 200 ml Wasser gelöst. Dann wird eine kalte Lösung von 110 g konz. Schwefelsäure in 200 ml Wasser unter Rühren zugetropft und danach das Wasser bei Normaldruck abdestilliert, bis eine Innentemperatur von 115°C erreicht ist. Dann wird im Wasserstrahlvakuum bis zu einer Badtemperatur von 180°C weiter destilliert und der Kolbeninhalt unter diesen Bedingungen so lange gehalten, bis er fest wird. Anschließend wird bei Wasserstrahlvakuum und 180°C noch 1 Stunde weiter erhitzt.

Das Produkt wird mit einer Lösung von 100 g NaOH in 200 ml Wasser versetzt, zerkleinert und über Nacht stehengelassen. Von der Suspension wird unter Normaldruck Wasser abdestilliert. Zu dem Rückstand werden 50 g KOH unter Rühren zugegeben und 3 Stunden gerührt. Nach Stehenlassen wird die obere Schicht abgetrennt, mit 20 g KOH versetzt und über Nacht stehengelassen. Das Öl wird abgegossen, mit 10 g KOH versetzt und unter Normaldruck destilliert.
Ausbeute: 60,3 g

b) (2-Hydroxy-ethyl)-(2-amino-2-methyl-propyl)-sulfid

234 g 2-Mercaptoethanol werden in 90 ml Dimethoxyethan gelöst. Zu dieser Lösung werden 63,3 g 2,2-Dimethylaziridin zugetropft und 4 Stunden bei 70°C gerührt, eingeengt und im Wasserstrahlvakuum destilliert.
Kp.: 141-142°C (bei 20 mbar) Fp.: 49-51°

c) 3,3-Dimethyl-tetrahydro-1,4-thiazin

Zu einer Lösung von 14,9 g (2-Hydroxy-ethyl)-(2-amino-2-methyl-propyl)-sulfid (Stufe b) in 100 ml Dimethoxyethan wird unter Rühren bis zur Sättigung Chlorwasserstoff eingeleitet und dann 17,9 g Thionylchlorid zugetropft und anschließend 1 Stunde bei Raumtemperatur weitergerührt und danach 1 Stunde am Rückfluß erhitzt. Die Lösung wird dann im Wasserstrahlvakuum eingeengt. Der Rückstand (2-Chloro-ethyl)-(2-amino-2-methyl-propyl)-sulfid-hydrochlorid kristallisiert aus.

Das erhaltene Hydrochlorid wird in Wasser gelöst, mit Pottasche stark alkalisch gestellt, in 100 ml Toluol aufgenommen und die wäßrige Phase nochmals mit 2 x 30 ml Toluol ausgeschüttelt. Die organischen Phasen werden vereinigt, über Pottasche getrocknet, eingeengt und im Wasserstrahlvakuum destilliert.
Ausbeute: 8,1 g Kp.: 73-75°C (bei 20 mbar)

d) 4-Nitroso-3,3-dimethyl-tetrahydro-1,4-thiazin

Eine Mischung von 15,7 g 3,3-Dimethyl-tetrahydro-1,4-thiazin (Stufe c) und 60 ml Eiswasser werden unter Kühlung mit 12 ml 10 n HCl versetzt. Dann wird bei 4 bis 6°C eine Lösung von 12,4 g Natriumnitrit in 40 ml Wasser zugetropft. Man rührt bei Raumtemperatur 4 Stunden nach, stellt mit Pottasche alkalisch und schüttelt mit Ethylacetat aus. Die organische Phase wird getrocknet und im Wasserstrahlvakuum eingeengt.
Ausbeute: 17,5 g Kp.: Gelbes Öl

e) 4-Amino-3,3-dimethyl-tetrahydro-1,4-thiazin-hydrochlorid

Eine Lösung von 17,2 g 4-Nitroso-3,3-dimethyl-tetrahydro-1,4-thiazin (Stufe d) in 150 ml Tetrahydrofuran wird bei 60-65°C portionsweise mit 4,4 g Lithiumaluminiumhydrid versetzt und danach 2 Stunden am Rückfluß erhitzt. Die Suspension wird abgekühlt und eine Lösung von 5 ml Wasser in 50 ml Tetrahydrofuran zugetropft. Nach 20 Minuten werden 10 ml 27gew.%ige Natronlauge zugetropft und einige Stunden stehen gelassen. Dann wird abfiltriert und im eisgekühlten Filtrat das Hydrochlorid gefällt.
Ausbeute: 12,2 g

f) 4-(2-Cyanoethyl-amino)-3,3-dimethyl-tetrahydro-1,4-thiazin

Zu einer auf -3 bis -5°C gekühlten Lösung von 12,1 g 4-Amino-3,3-dimethyl-tetrahydro-1,4-thiazin-hydrochlorid (Stufe e) wird eine Lösung von 6,1 g KCN in 30 ml Wasser zugetropft. Dann wird mit Salzsäure der pH Wert der Lösung auf 7,2 gestellt und danach 16,3 g 39gew.%iges wäßriges Formaldehyd zugesetzt,

wobei der pH Wert auf 6 bis 7 gehalten wird. Es wird über Nacht bei auf Raumtemperatur ansteigende Temperatur nachgerührt. Die Suspension wird mit Ethylacetat ausgeschüttelt, die organische Phase getrocknet und eingeengt.
Ausbeute: 9,4 g Kp.: Öl

g) 3-(3,3-Dimethyl-tetrahydro-1,4-thiazin-4-yl)-sydnonimin-hydrochlorid

9,4 g 4-(2-Cyanoethyl-amino)-3,3-dimethyl-tetrahydro-1,4-thiazin (Stufe f), 40 ml Ethylacetat, 40 g Eiswasser und 5 ml 10 n Salzsäure werden auf -5°C gekühlt. In diese Mischung werden 5,3 g Natriumnitrit eingetragen und anschließend 3 Stunden bei Raumtemperatur gerührt. Die organische Phase wird abgetrennt, getrocknet, abfiltriert und unter Kühlen 20 ml einer gesättigten Lösung von HCl in Isopropanol zugetropft. Es wird über Nacht bei Raumtemperatur nachgerührt. Der Niederschlag wird abgetrennt und aus Isopropanol umkristallisiert.
Ausbeute: 5,9 g Kp.: 172°C (Zersetzung)

Beispiel 6

N-p-Anisoyl-3-(3,3-dimethyl-tetrahydro-1,4-thiazin-4-yl)-sydnonimin

2 g 3-(3,3-Dimethyl-tetrahydro-1,4-thiazin-4-yl)-sydnonimin-hydrochlorid (Beispiel 5 g) werden in 20 ml Eiswasser gelöst und 1,7 g $NaHCO_3$ zugegeben. Dann werden 1,7 g p-Anisoylchlorid, gelöst in 20 ml Ethylacetat, zugetropft. Nach dem Nachrühren über Nacht bei Raumtemperatur werden die Phasen getrennt, die wäßrige Phase mit Ethylacetat ausgeschüttelt, die organischen Phasen vereinigt, getrocknet und eingeengt. Das erhaltene kristalline Produkt wird aus Isopropanol umkristallisiert.
Ausbeute: 1,9 g Kp.: 131 bis 133°C

Beispiel 7

N-Ethoxycarbonyl-3-(3,3-dimethyl-tetrahydro-1,4-thiazin-4-yl)-sydnonimin

Die Verbindung wird analog Beispiel 6 mit Chlorameisensäureethylester (1 g) anstelle von p-Anisoylchlorid erhalten und durch Verrühren mit Petrolether gereinigt.
Ausbeute: 1,8 g Kp.: 110 bis 112°C

Beispiel 8

N-Methoxycarbonyl-3-(3,3-dimethyl-1,4-tetrahydro-thiazin-1,1-dioxo-4-yl)-sydnonimin

Die Verbindung wird analog Beispiel 7 mit Chlorameisensäuremethylester (0,6 g) anstelle des Ethylesters erhalten und aus Ethylacetat umkristallisiert.
Ausbeute: 2,0 g Kp.: 233 bis 235°C (Zers.)

Beispiel 9

N-Butoxycarbonyl-3-(3,3-dimethyl-1,4-tetrahydro-thiazin-1,1-dioxo-4-yl)-sydnonimin-hydrochlorid

Die Verbindung wird analog Beispiel 7 mit Chlorameisensäurebutylester (0,8 g) anstelle des Ethylester erhalten und aus Ethylacetat mit isopropanolischer Salzsäure als Hydrochlorid gefällt.
Ausbeute: 1,3 g Kp.: 153 bis 154°C (Zers.)

Beispiel 10

N-Butoxyacetyl-3-(3,3-dimethyl-1,4-tetrahydro-thiazin-1,1-dioxo-4-yl)-sydnonimin-hydrochlorid

Die Verbindung wird analog Beispiel 7 mit 0,9 g Butoxyacetylchlorid anstelle von Chlorameisensäurethylester erhalten und aus Essigester mit isopropanolischer Salzsäure als Hydrochlorid gefällt.
Ausbeute: 1,9 g Kp.: 165°C (Zers.)

Beispiel 11

3-(3,3-Dimethyl-1,4-tetrahydro-thiazin-1,1-dioxid-4-yl)-sydnonimin-hydrochlorid

Die Lösung von 3 g 3-(3,3-Dimethyl-tetrahydro-1,4-thiazin-4-yl)-sydnonimin (hergestellt nach Beispiel 5) in 40 ml Eisessig wird bei Raumtemperatur mit 3 g einer 35%igen Wasserstoffperoxidlösung versetzt. Die Temperatur steigt auf 30 bis 40°C an. Die Mischung wird 2 h gerührt und dann im Wasserstrahlvakuum eingeengt. Der Rückstand wird in Isopropanol verrührt und aus Methanol umkristallisiert.
Ausbeute: 1,8 g Kp.: 219°C (Zers.)

In den nachfolgenden Beispielen A bis H werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

Beispiel E

Tabletten, enthaltend 2 mg Wirkstoff pro Tablette

| | pro Tablette |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

Beispiel G

Für die Herstellung des Inhalts von Hartgelatinekapseln eignen sich die folgenden Rezepturen:

| | |
|---|---|
| a) Wirkstoff | 10 mg |
| Maisstärke | 190 mg |
| | 200 mg |

| | |
|---|---|
| b) Wirkstoff | 40 mg |
| Milchzucker | 80 mg |
| Maisstärke | 80 mg |
| | 200 mg |

Beispiel H

Tropfen können nach folgender Rezeptur hergestellt werden (20 mg Wirkstoff in 1 ml = 20 Tropfen):

| | |
|---|---|
| Wirkstoff | 2,00 g |
| Benzoesäuremethylester | 0,07 g |
| Benzoesäureethylester | 0,03 g |
| Ethanol 96%ig | 4 ml |
| entmineralisiertes Wasser ad | 100 ml |

## Patentansprüche

### Patentansprüche für folgende Vertragsstaaten : BE, DE, DK, FR, GB, IT, NL, AT, SE, CH, LI

1. Substituierte 3-Aminosydnonimine der allgemeinen Formel I

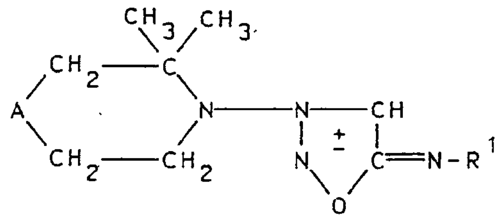

(I)

und ihre pharmakologisch annehmbaren Säureadditionssalze, worin
A den Rest -S- oder $-SO_2-$,
$R^1$ den Rest $-CO-R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten.

2. Substituierte 3-Aminosydnonimine nach Anspruch 1, dadurch gekennzeichnet, daß $R^2$ ($C_1$ bis $C_4$)Alkyl, ($C_1$ bis $C_4$)Alkoxy, Phenyl, oder durch Chlor, Methyl oder Methoxy monosubstituiertes Phenyl bedeutet.

3. Die pharmakologisch annehmbaren Säureadditionssalze, insbesondere das Hydrochlorid der Verbindung 3-(3,3-Dimethyl-tetrahydro-1,4-thiazin-4-yl)-sydnonimin.

4. N-p-Anisoyl-3-(3,3-dimethyl-tetrahydro-1,4-thiazin-4-yl)-sydnonimin und seine pharmakologisch annehmbaren Säureadditionssalze, insbesondere sein Hydrochlorid.

5. Die pharmakologisch annehmbaren Säureadditionssalze, insbesondere das Hydrochlorid der Verbindung 3-(3,3-Dimethyl)-1,4-tetrahydrothiazin-1,1-dioxid-4-yl)-sydnonimin.

6. Verfahren zur Herstellung der in Anspruch 1 und/oder 2 angegebenen Verbindungen der Formel I und/oder der in einem oder mehreren der Ansprüche 3 bis 5 angegebenen Verbindungen, dadurch gekennzeichnet, daß eine Verbindung der allgemeinen Formel II

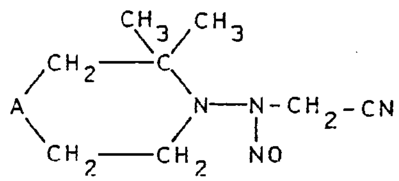

(II)

worin A den Rest -S- oder -$SO_2$- bedeutet, zu einer Verbindung der Formel Ia

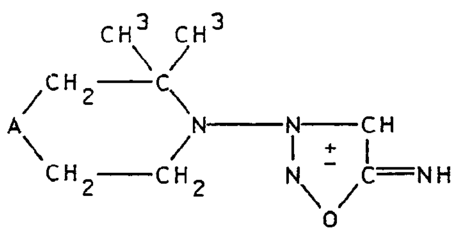

(Ia)

cyclisiert und die erhaltene Verbindung als Säureadditionssalz isoliert wird, oder daß für den Fall, daß eine Verbindung der Formel I mit $R^1$ = -$COR^2$ hergestellt werden soll, die Verbindung Ia oder ein Säureadditionssalz davon mit einem Acylierungsmittel, das den Rest -$COR^2$ einführt, acyliert wird und die so erhaltene Verbindung isoliert oder gegebenenfalls in ein pharmakologisch annehmbares Säureadditionssalz überführt, und dieses isoliert wird.

7. Verfahren nach Anspruch 6, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

8. Verfahren zur Herstellung substituierter 3-Aminosydnoniminen der allgemeinen Formel Ib

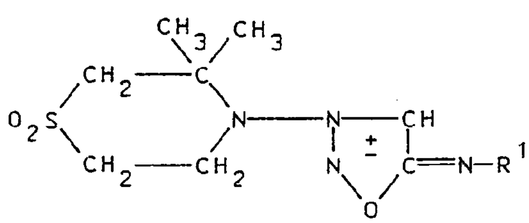

(Ib)

und ihrer pharmakologisch annehmbaren Säureadditionssalze, worin
$R^1$ Wasserstoff oder den Rest -$COR^2$,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)-Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest
bedeuten, dadurch gekennzeichnet, daß eine Verbindung der allgemeinen Formel Ic

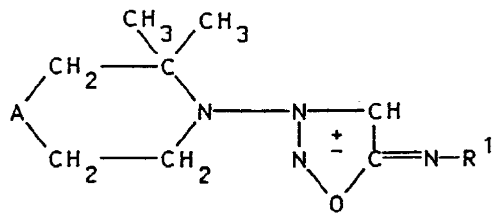

(Ic)

worin
A den Rest -S- oder -SO- bedeutet und
$R^1$ die bereits genannte Bedeutung hat,
oder ein Säureadditionssalz davon mit einer Sauerstoff abgebenden Verbindung umgesetzt und die erhaltene Verbindung gegebenenfalls in ein Säureadditionssalz überführt und isoliert wird, oder daß für den Fall, daß eine Verbindung der Formel I mit A = $-SO_2-$ und $R^1$ = H erhalten worden ist und eine Verbindung der Formel I mit A = $-SO_2-$ und $R^1$ = $-COR^2$ hergestellt werden soll, die erhaltene Verbindung oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt und dieses isoliert wird.

**9.** 3-Amino-sydnonimine und ihre pharmakologisch annehmbaren Säureadditionssalze eines oder mehrerer der Ansprüche 1 bis 5 als pharmakologische Wirkstoffe zur Anwendung bei der Bekämpfung und Vorbeugung cardiovaskulärer Erkrankungen oder als pharmazeutische Wirkstoffe zur Herstellung pharmazeutischer Präparate.

**10.** Pharmazeutisches Präparat, dadurch gekennzeichnet, daß es eine oder mehrere Verbindungen der Ansprüche 1 bis 5 oder ein oder mehrere Säureadditionssalze davon als Wirkstoff oder Wirkstoffe zusammen mit einem oder mehreren pharmazeutisch annehmbaren Träger-und Zusatzstoffen und gegebenenfalls noch eine oder mehrere andere pharmakologische Wirkstoffe enthält.

**Patentansprüche für folgenden Vertragsstaat : ES**

**1.** Verfahren zur Herstellung substituierter 3-Aminosydnonimine der allgemeinen Formel I

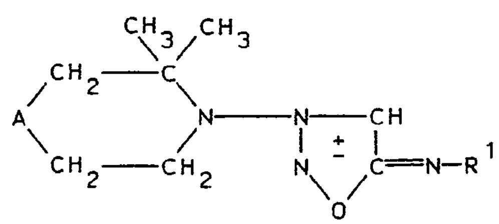

(I)

und ihrer pharmakologisch annehmbarer Säureadditionssalze,
worin
A den Rest - S- oder $-SO_2-$,
$R^1$ den Rest $-CO-R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten 1 bis 4 C-Atomen und/oder 1 bis 3 Alkoxyresten mit 1 bis 4 C-Atomen mono-, di- oder trisubstituierten Phenylrest bedeuten, dadurch gekennzeichnet, daß eine Verbindung der allgemeinen Formel II

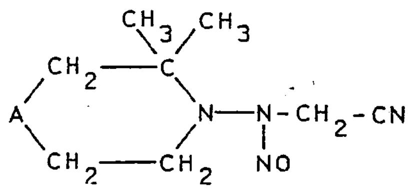

(II)

worin A den Rest -S- oder $-SO_2-$ bedeutet, zu einer Verbindung der Formel Ia

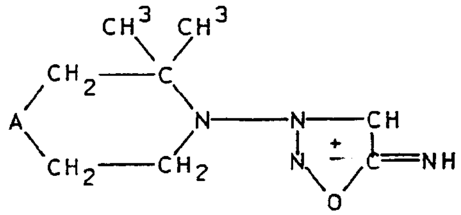

(Ia)

cyclisiert und die erhaltene Verbindung als Säureadditionssalz isoliert wird, oder daß für den Fall, daß eine Verbindung der Formel I mit $R^1$ = $-COR^2$ hergestellt werden soll, die Verbindung Ia oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt, und dieses isoliert wird.

2. Verfahren nach Anspruch 1, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

3. Verfahren nach Anspruch 1 und/oder 2, dadurch gekennzeichnet, daß mit einem Acylierungsmittel acyliert wird, das den Rest $-CO-R^2$ einführt, wobei $R^2$ ($C_1$ bis $C_4$)Alkyl, ($C_1$ bis $C_4$)Alkoxy, Phenyl, oder durch Chlor, Methyl oder Methoxy monosubstituiertes Phenyl bedeutet.

4. Verfahren nach Anspruch 1 und/oder 2, dadurch gekennzeichnet, daß die Ausgangsverbindungen so ausgewählt werden, daß ein pharmakologisch annehmbares Säureadditionssalz, insbesondere das Hydrochlorid der Verbindung 3-(3,3-Dimethyl-tetrahydro-1,4-thiazin-4-yl)-sydnonimin entsteht.

5. Verfahren nach Anspruch 1 und/oder 2, dadurch gekennzeichnet, daß die Ausgangsverbindungen so ausgewählt werden, daß N-p-Anisoyl-3-(3,3-dimethyl-tetrahydro-1,4-thiazin-4-yl)-sydnonimin oder ein pharmakologisch annehmbares Säureadditionssalz davon, insbesondere sein Hydrochlorid, entsteht.

6. Verfahren nach Anspruch 1 und/oder 2, dadurch gekennzeichnet, daß die Ausgangsverbindungen so ausgewählt werden, daß ein pharmakologisch annehmbares Säureadditionssalz, insbesondere das Hydrochlorid der Verbindung 3-(3,3-Dimethyl)-1,4-tetrahydrothiazin-1,1-dioxid-4-yl)-sydnonimins entsteht.

7. Verfahren zur Herstellung substituierter 3-Aminosydnoniminen der allgemeinen Formel Ib

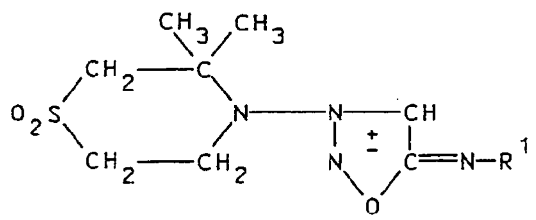

(Ib)

und ihrer pharmakologisch annehmbaren Säureadditionssalze, worin
$R^1$ Wasserstoff oder den Rest $-COR^2$,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)-Cycloalkyl, Phenyl,

einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, dadurch gekennzeichnet, daß eine Verbindung der allgemeinen Formel Ic

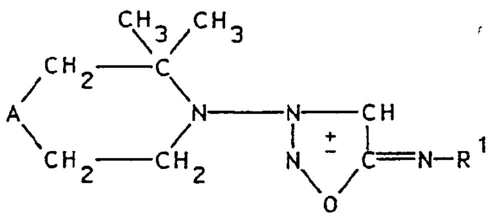

(Ic)

worin
A den Rest -S- oder -SO- bedeutet und
$R^1$ die bereits genannte Bedeutung hat,
oder ein Säureadditionssalz davon mit einer Sauerstoff abgebenden Verbindung umgesetzt und die erhaltene Verbindung gegebenenfalls in ein Säureadditionssalz überführt und isoliert wird, oder daß für den Fall, daß eine Verbindung der Formel I mit A = $-SO_2-$ und $R^1$ = H erhalten worden ist und eine Verbindung der Formel I mit A = $-SO_2-$ und $R^1$ = $-COR^2$ hergestellt werden soll, die erhaltene Verbindung oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt und dieses isoliert wird.

8. Verfahren nach Anspruch 7, dadurch gekennzeichnet, daß die Ausgangsverbindungen so ausgewählt werden, daß ein pharmakologisch annehmbares Säureadditionssalz, insbesondere das Hydrochlorid der Verbindung 3-(3,3-Dimethyl)-1,4-tetrahydrothiazin-1,1-dioxid-4-yl)-sydnonimin entsteht.

9. Verwendung von substituierten 3-Aminosydnoniminen der allgemeinen Formel I

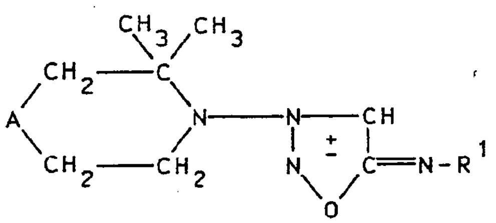

(I)

und ihrer pharmakologisch annehmbaren Säureadditionssalze, worin
A den Rest -S- oder $-SO_2-$,
$R^1$ den Rest $-CO-R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, als pharmakologische Wirkstoffe zur Herstellung pharmazeutischer Präparate.

10. Verfahren zur Herstellung eines Pharmazeutischen Präparates, dadurch gekennzeichnet, daß ein oder mehrere substituierte 3-Aminosydnonimine der allgemeinen Formel I

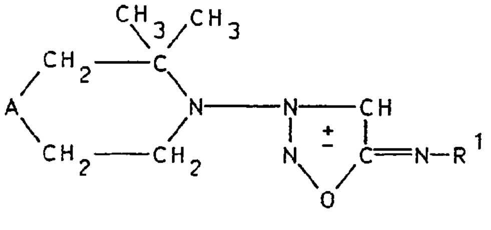

(I)

und/oder ein oder mehrere pharmakologisch annehmbaren Säureadditionssalze davon, worin
A den Rest -S- oder -$SO_2$-,
$R^1$ den Rest -CO-$R^2$ oder Wasserstoff
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, als Wirkstoff oder Wirkstoffe zusammen mit einem oder mehreren pharmazeutisch annehmbaren Träger- und/oder Zusatzstoffen und gegebenenfalls noch einem oder mehreren anderen pharmakologischen Wirkstoffen in an sich bekannter Weise in eine zur Verabreichung geeignete pharmazeutische Zubereitung überführt werden.

## Claims

**Claims for the following Contracting States : BE, DE, DK, FR, GB, IT, NL, AT, SE, CH, LI**

1. Substituted 3-aminosydnone imines of the general formula I

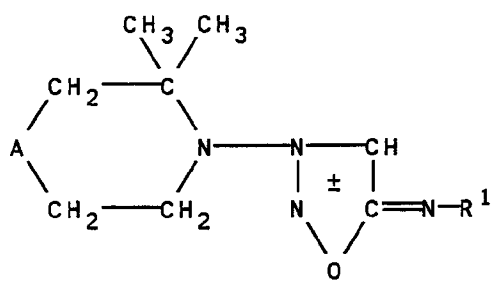

(I)

and their pharmacologically acceptable acid addition salts, in which
A denotes the radical -S-, or $SO_2$-,
$R^1$ denotes the radical -CO-$R^2$ or hydrogen,
$R^2$ denotes ($C_1$ to $C_4$)alkyl, ($C_1$ to $C_4$)alkoxy-($C_1$ to $C_4$)alkyl, ($C_1$ to $C_4$)alkoxy, ($C_5$ to $C_7$)cycloalkyl, phenyl, or a phenyl radical which is mono, di- or trisubstituted by 1 to 3 halogen atoms and/or 1 to 3 alkyl radicals having 1 to 4 C atoms and/or 1 to 3 alkoxy radicals having 1 to 4 C atoms.

2. Substituted 3-aminosydnone imines according to Claim 1, characterized in that $R^2$ denotes ($C_1$ to $C_4$)alkyl, ($C_1$ to $C_4$)alkoxy, phenyl, or phenyl monosubstituted by chlorine, methyl or methoxy.

3. The pharmacologically acceptable acid addition salts, in particular the hydrochloride, of the compound 3-(3,3-dimethyltetrahydro-1,4-thiazin-4-yl)sydnone imine.

4. N-p-Anisoyl-3-(3,3-dimethyltetrahydro-1,4-thiazin-4-yl)sydnone imine and its pharmacologically acceptable acid addition salts, in particular its hydrochloride.

5. The pharmacologically acceptable acid addition salts, in particular the hydrochloride, of the compound 3-(3,3-dimethyl)-1,4-tetrahydrothiazine-1,1-dioxide-4-yl)sydnone imine.

6. Process for the preparation of the compounds of the formula I indicated in Claim 1 and/or 2 and/or the compounds indicated in one or more of Claims 3 to 5, characterized in that a compound of the general formula II

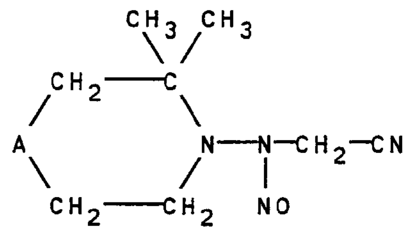

(II)

in which A denotes the radical -S- or $-SO_2-$, is cyclized to give a compound of the formula Ia

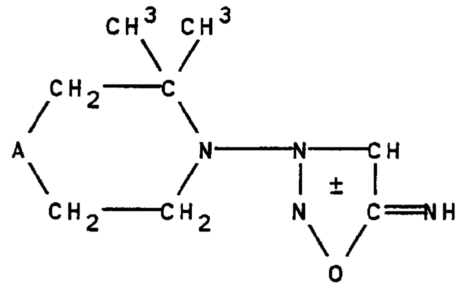

(Ia)

and the compound obtained is isolated as the acid addition salt, or in that in the case in which it is intended to prepare a compound of the formula I where $R^1 = -COR^2$, the compound Ia or an acid addition salt thereof is acylated with an acylating agent which introduces the radical $-COR^2$ and the compound thus obtained is isolated or, if desired, converted into a pharmacologically acceptable acid addition salt, and this is isolated.

7. Process according to Claim 6, characterized in that the cyclization is carried out in a solvent, dispersant or diluent at temperatures of -10 to 40°C, preferably 0 to 20°C, with the aid of a cyclizing agent which establishes a pH below 3 in aqueous solution.

8. Process for the preparation of substituted 3-amino-sydnone imines of the general formula Ib

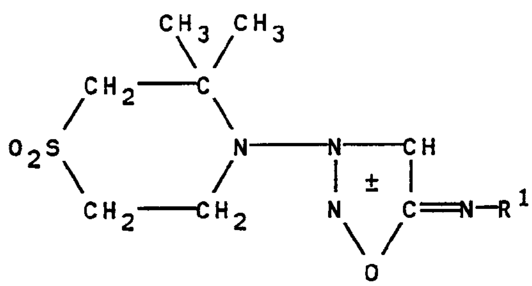

(Ib)

and their pharmacologically acceptable acid addition salts, in which
$R^1$ denotes hydrogen or the radical $-COR^2$,
$R^2$ denotes ($C_1$ to $C_4$)alkyl, ($C_1$ to $C_4$)alkoxy-($C_1$ to $C_4$)alkyl, ($C_1$ to $C_4$)alkoxy, ($C_5$ to $C_7$)cycloalkyl, phenyl, or a phenyl radical which is mono-, di- or trisubstituted by 1 to 3 halogen atoms and/or 1 to 3 alkyl radicals having 1 to 4 C atoms and/or 1 to 3 alkoxy radicals having 1 to 4 C atoms, characterized in that a compound of the general formula Ic

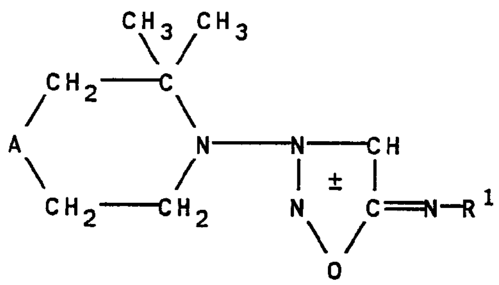

(Ic)

in which
A denotes the radical -S- or -SO- and
$R^1$ has the meaning already mentioned, or an acid addition salt thereof is reacted with an oxygen-donating compound and, if desired, the compound obtained is converted into an acid addition salt and isolated, or in that, in the case in which a compound of the formula I where A = $-SO_2-$and $R^1$ = H has been obtained and it is intended to prepare a compound of the formula I where A = $-SO_2-$ and $R^1$ = $-COR^2$, the compound obtained or an acid addition salt thereof is acylated with an acylating agent which introduces the radical $-COR^2$ and the compound thus obtained is isolated or, if desired, converted into a pharmacologically acceptable acid addition salt and this is isolated.

**9.** 3-Aminosydnone imines and their pharmacologically acceptable acid addition salts of one or more of Claims 1 to 5 as pharmacological active compounds for use in the combating and prevention of cardiovascular disorders or as pharmaceutical active compounds for the production of pharmaceutical preparations.

**10.** Pharmaceutical preparation, characterized in that it contains one or more compounds of one of Claims 1 to 5 or one or more acid addition salts thereof as active compound or active compounds together with one or more pharmaceutically acceptable excipients and additives and, if desired, also one or more other pharmacological active compounds.

**Claims for the following Contracting State : ES**

**1.** Process for preparing substituted 3-aminosydnone imines of the general formula I

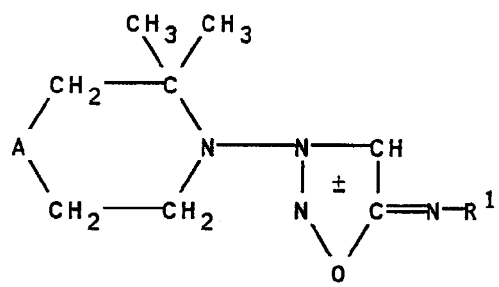

(I)

and their pharmacologically acceptable acid addition salts, in which
A denotes the radical -S-, or $SO_2$-,
$R^1$ denotes the radical $-CO-R^2$ or hydrogen,
$R^2$ denotes ($C_1$ to $C_4$)alkyl, ($C_1$ to $C_4$)alkoxy-($C_1$ to $C_4$)alkyl, ($C_1$ to $C_4$)alkoxy, ($C_5$ to $C_7$)cycloalkyl, phenyl, or a phenyl radical which is mono, di- or trisubstituted by 1 to 3 halogen atoms and/or 1 to 3 alkyl radicals having 1 to 4 C atoms and/or 1 to 3 alkoxy radicals having 1 to 4 C atoms, characterized in that a compound of the general formula II

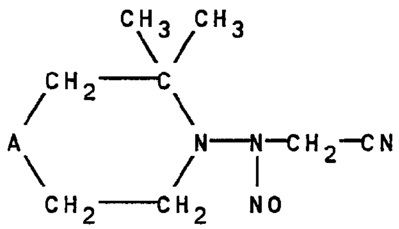

(II)

in which A denotes the radical -S- or $-SO_2-$, is cyclized to give a compound of the formula Ia

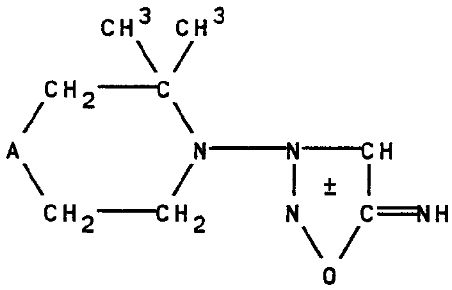

(Ia)

and the compound obtained is isolated as the acid addition salt, or in that in the case in which it is intended to prepare a compound of the formula I where $R^1 = -COR^2$, the compound Ia or an acid addition salt thereof is acylated with an acylating agent which introduces the radical $-COR^2$ and the compound thus obtained is isolated or, if desired, converted into a pharmacologically acceptable acid addition salt, and this is isolated.

2. Process according to Claim 1, characterized in that the cyclization is carried out in a solvent, dispersant or diluent at temperatures of -10 to 40°C, preferably 0 to 20°C, with the aid of a cyclizing agent which establishes a pH below 3 in aqueous solution.

3. Process according to Claim 1 and/or 2, characterized in that the acylation is carried out with an acylating agent which introduces the radical $-CO-R^2$, in which $R^2$ denotes ($C_1$ to $C_4$)alkyl, ($C_1$ to $C_4$)alkoxy, phenyl or phenyl monosubstituted by chlorine, methyl or methoxy.

4. Process according to Claim 1 and/or 2, characterized in that the starting compounds are chosen such that a pharmacologically acceptable acid addition salt, in particular the hydrochloride of the compound 3-(3,3-dimethyl-tetrahydro-1,4-thiazin-4-yl)-sydnone imine is formed.

5. Process according to Claim 1 and/or 2, characterized in that the starting compounds are chosen such that N-p-anisoyl-3-(3,3-dimethyl-tetrahydro-1,4-thiazin-4-yl)sydnone imine or a pharmacologically acceptable acid addition salt, in particular its hydrochloride, is formed.

6. Process according to Claim 1 and/or 2, characterized in that the starting compounds are chosen such that a pharmacologically acceptable acid addition salt, in particular the hydrochloride of the compound 3-(3,3-dimethyl)-1,4-tetrahydrothiazin-1,1-dioxide-4-yl)sydnone imine, is formed.

7. Process for the preparation of substituted 3-amino -sydnone imines of the general formula Ib

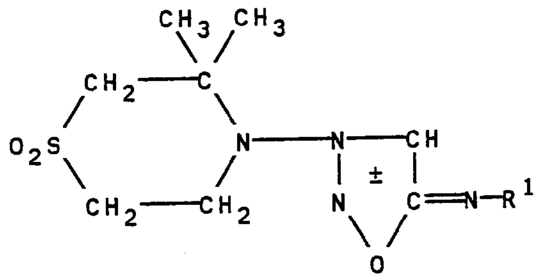

(Ib)

and their pharmacologically acceptable acid addition salts, in which
$R^1$ denotes hydrogen or the radical $-COR^2$,
$R^2$ denotes ($C_1$ to $C_4$)alkyl, ($C_1$ to $C_4$)alkoxy-($C_1$ to $C_4$)alkyl, ($C_1$ to $C_4$)alkoxy, ($C_5$ to $C_7$)cycloalkyl, phenyl, or a phenyl radical which is mono-, di- or trisubstituted by 1 to 3 halogen atoms and/or 1 to 3 alkyl radicals having 1 to 4 C atoms and/or 1 to 3 alkoxy radicals having 1 to 4 C atoms, characterized in that a compound of the general formula Ic

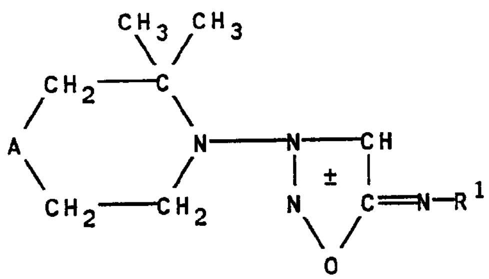

(Ic)

in which
A denotes the radical -S- or -SO- and
$R^1$ has the meaning already mentioned, or an acid addition salt thereof is reacted with an oxygen-donating compound and, if desired, the compound obtained is converted into an acid addition salt and isolated, or in that, in the case in which a compound of the formula I where A = $-SO_2-$ and $R^1$ = H has been obtained and it is intended to prepare a compound of the formula I where A = $-SO_2-$ and $R^1$ = $-COR^2$, the compound obtained or an acid addition salt thereof is acylated with an acylating agent which introduces the radical $-COR^2$ and the compound thus obtained is isolated or, if desired, converted into a pharmacologically acceptable acid addition salt and this is isolated.

8. Process according to Claim 7, characterized in that the starting compounds are chosen such that a pharmacologically acceptable acid addition salt, in particular the hydrochloride of the compound 3-(3,3-dimethyl)1,4-tetrahydrothiazin-1,1-dioxide-4-yl)sydnone imine is formed.

9. Use of substituted 3-aminosydnone imines of the general formula I

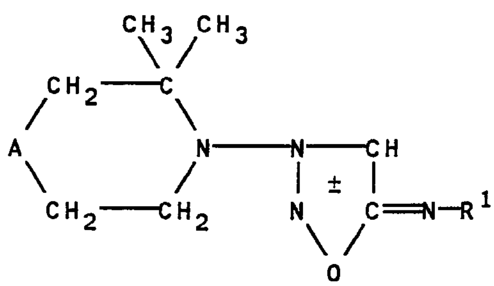

(I)

and their pharmacologically acceptable acid addition salts, in which
A denotes the radical -S- or $SO_2$-,

$R^1$ denotes the radical -CO-$R^2$ or hydrogen,
$R^2$ denotes ($C_1$ to $C_4$)alkyl, ($C_1$ to $C_4$)alkoxy-($C_1$ to $C_4$)alkyl, ($C_1$ to $C_4$)alkoxy, ($C_5$ to $C_7$)cycloalkyl, phenyl, or a phenyl radical which is mono, di- or trisubstituted by 1 to 3 halogen atoms and/or 1 to 3 alkyl radicals having 1 to 4 C atoms and/or 1 to 3 alkoxy radicals having 1 to 4 C atoms, as pharmacological active compounds for preparing pharmaceutical preparations.

**10.** Process for preparing a pharmaceutical preparation, characterized in that one or more substituted 3-aminosydnone imines of the general formula I

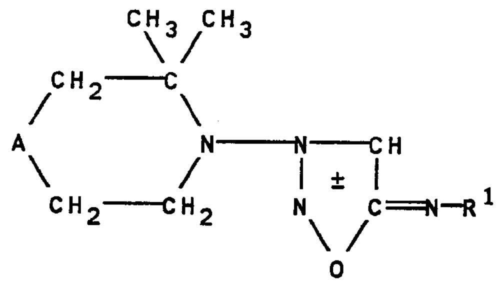

(I)

and/or one or more pharmacologically acceptable acid addition salts, in which
A denotes the radical -S- or $SO_2$-,
$R^1$ denotes the radical -CO-$R^2$ or hydrogen,
$R^2$ denotes ($C_1$ to $C_4$)alkyl, ($C_1$ to $C_4$)alkoxy-($C_1$ to $C_4$)alkyl, ($C_1$ to $C_4$)alkoxy, ($C_5$ to $C_7$)cycloalkyl, phenyl, or a phenyl radical which is mono, di- or trisubstituted by 1 to 3 halogen atoms and/or 1 to 3 alkyl radicals having 1 to 4 C atoms and/or 1 to 3 alkoxy radicals having 1 to 4 C atoms are converted in a manner known by itself as active compound or active compounds together with one or more pharmaceutically acceptable excipients and/or additives and, if desired, also one or more other pharmacological active compounds, into a pharmaceutical preparation which is suitable for administration.

## Revendications

**Revendications pour les Etats contractants suivants : BE, DE, DK, FR, GB, IT, NL, AT, SE, CH, LI**

**1.** 3-aminosydnonimines substituées qui répondent à la formule générale I :

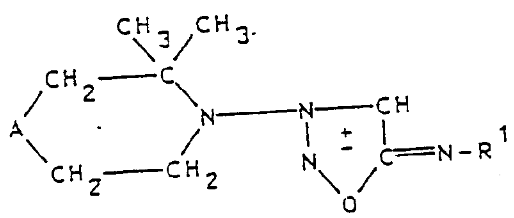

(I)

dans laquelle :

A représente un radical -S- ou -$SO_2$-,
$R^1$ représente un radical -CO-$R^2$ ou l'hydrogène, et
$R^2$ représente un alkyle en $C_1$-$C_4$, un ($C_1$-$C_4$)alcoxy($C_1$-$C_4$)alkyle, un alcoxy en $C_1$-$C_4$, un cycloalkyle en $C_5$-$C_7$, un phényle ou un phényle monosubstitué, disubstitué ou trisubstitué qui porte de 1 à 3 atomes d'halogènes et/ou de 1 à 3 radicaux alkyles contenant de 1 à 4 atomes de carbone et/ou de 1 à 3 radicaux alcoxy contenant de 1 à 4 atomes de carbone,

ainsi que les sels d'addition, acceptables du point de vue pharmacologique, qu'elles forment avec des acides.

**2.** 3-aminosydnonimines substituées selon la revendication 1, caractérisées en ce que $R^2$ représente un alkyle en $C_1$-$C_4$, un alcoxy en $C_1$-$C_4$, un phényle ou un phényle monosubstitué qui porte un atome de chlore

ou un radical méthyle ou méthoxy.

3. Sels d'addition d'acides acceptables du point de vue pharmacologique, en particulier chlorhydrate de la 3-(3,3-diméthyl-tétrahydro-1,4-thiazine-4-yl)sydnonimine.

4. N-p-anisoyl-3-(3,3-diméthyl-tétrahydro-1,4-thiazine-4-yl)-sydnonimine et ses sels d'addition d'acides acceptables du point de vue pharmacologique, en particulier son chlorhydrate.

5. Sels d'addition d'acides acceptables du point de vue pharmacologique, en particulier chlorhydrate, de la 3-(3,3-diméthyl-tétrahydro-1,4-thiazine-1,1-dioxyde-4-yl)-sydnonimine.

6. Procédé pour préparer les composés de formule I définis dans les revendications 1 et/ou 2, et/ou des composés cités dans une ou plusieurs des revendications 3 à 5, procédé caractérisé en ce qu'on cyclise un composé répondant à la formule générale II :

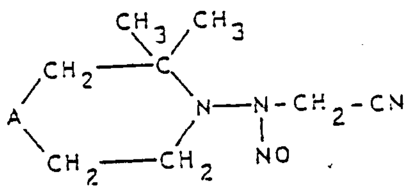

(II)

dans laquelle A représente un radical -S- ou $-SO_2-$, de manière à le convertir en un composé de formule Ia :

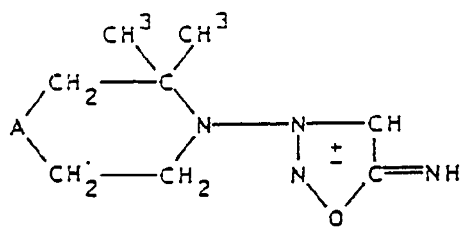

(Ia)

et on isole le composé obtenu à l'état de sel d'addition d'acide, ou, dans le cas où l'on veut préparer un composé de formule I pour lequel $R^1$ représente un radical $-COR^2$, on acyle le composé Ia, ou un de sel d'addition d'acide de celui-ci, au moyen d'un agent d'acylation introduisant le radical $-COR^2$ et on isole le composé ainsi formé, ou on le transforme éventuellement en un sel d'addition d'acide acceptable du point de vue pharmacologique et on isole celui-ci.

7. Procédé selon la revendication 6, caractérisé en ce que la cyclisation est effectuée dans un solvant, dispersant ou diluant, à des températures comprises entre -10 et 40°C, de préférence entre 0 et 20°C, au moyen d'un agent de cyclisation qui, en solution aqueuse, donne au pH une valeur inférieure à 3.

8. Procédé pour préparer des 3-amino-sydnonimines substituées qui répondent à la formule générale Ib :

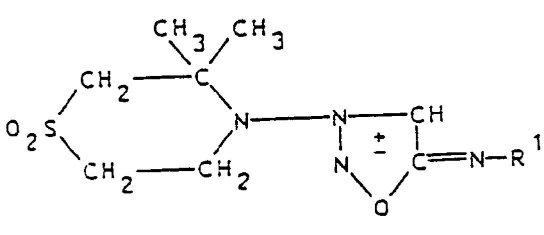

(Ib)

dans laquelle $R^1$ représente l'hydrogène ou un radical $-COR^2$, et $R^2$ représente un alkyle en $C_1$-$C_4$, un ($C_1$-$C_4$) alcoxy- ($C_1$-$C_4$) alkyle, un alcoxy en $C_1$-$C_4$, un cycloalkyle en $C_5$-$C_7$, un phényle ou un phényle mono-, di- ou tri-substitué qui porte de 1 à 3 atomes d'halogènes et/ou de 1 à 3 radicaux alkyles contenant chacun

de 1 à 4 atomes de carbone et/ou de 1 à 3 radicaux alcoxy contenant chacun de 1 à 4 atomes de carbone, et leurs sels d'addition d'acides acceptables du point de vue pharmacologique, procédé caractérisé en ce qu'on fait réagir un composé répondant à la formule générale Ic :

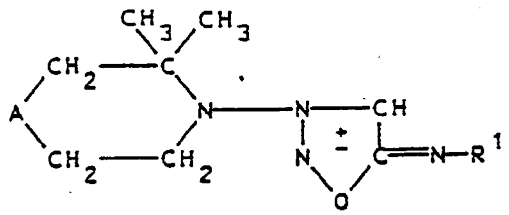

(Ic)

dans laquelle A représente un radical -S- ou -SO-et $R^1$ a la signification qui lui a été donnée ci-dessus, ou un sel d'addition d'acide d'un tel composé, avec un composé cédant de l'oxygène, on transforme éventuellement le composé ainsi obtenu en un sel d'addition d'acide et on l'isole, ou, dans le cas où l'on a obtenu un composé de formule I pour lequel A représente $-SO_2-$ et $R^1$ représente l'hydrogène et qu'on veut préparer un composé de formule I pour lequel A représente $-SO_2-$ et $R^1$ un radical $-COR^2$, on acyle le composé obtenu, ou un sel d'addition d'acide d'un tel composé, au moyen d'un agent d'acylation introduisant le radical $-COR^2$ et on isole le composé ainsi obtenu, ou on le transforme éventuellement en un sel d'addition d'acide acceptable du point de vue pharmacologique et on isole celui-ci.

**9.** 3-amino-sydnonimines et leurs sels d'addition d'acides acceptables du point de vue pharmacologique selon une ou plusieurs des revendications 1 à 5, en tant que substances actives pharmacologiques à utiliser pour le traitement curatif ou préventif de maladies cardiovasculaires ou comme substances actives pharmaceutiques pour la fabrication de compositions pharmaceutiques.

**10.** Composition pharmaceutique caractérisée en ce qu'elle contient un ou plusieurs composés selon les revendications 1 à 5, ou un ou plusieurs sels d'addition d'acides de tels composés, comme substance active ou substances actives, ainsi qu'un ou plusieurs excipients et additifs acceptables du point de vue pharmaceutique et, éventuellement, une ou plusieurs autres substances douées d'une activité pharmacologique.

**Revendications pour l'Etat contractant suivant : ES**

**1.** Procédé pour préparer des 3-amino-sydnonimines subsituées répondant à la formule générale I :

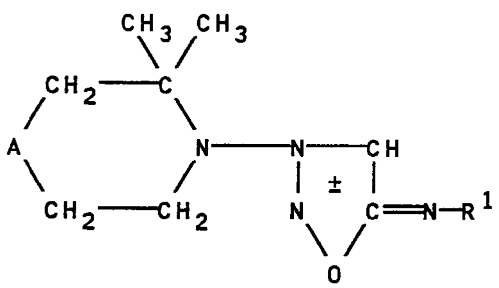

(I)

dans laquelle :

A représente un radical -S- ou $-SO_2-$,

$R^1$ représente un radical $-CO-R^2$ ou l'hydrogène, et

$R^2$ représente un alkyle en $C_1$-$C_4$, un ($C_1$-$C_4$)alcoxy-($C_1$-$C_4$)alkyle, un alcoxy en $C_1$-$C_4$, un cycloalkyle en $C_5$-$C_7$, un phényle ou un phényle monosubstitué, disubstitué ou trisubstitué qui porte de 1 à 3 atomes d'halogènes et/ou de 1 à 3 radicaux alkyles contenant de 1 à 4 atomes de carbone et/ou de 1 à 3 radicaux alcoxy contenant de 1 à 4 atomes de carbone, ainsi que les sels d'addition, acceptables du point de vue pharmacologique, qu'elles forment avec des acides, procédé caractérisé en ce qu'on cyclise un composé répondant à la formule générale II :

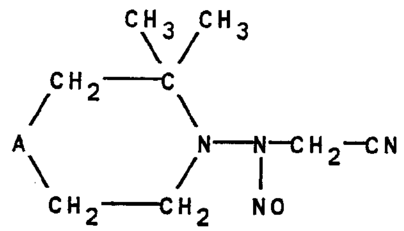

(II)

dans laquelle A représente un radical -S- ou $-SO_2$, de manière à le convertir en un composé de formule Ia :

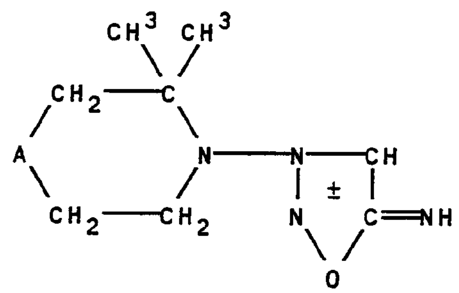

(Ia)

et on isole le composé obtenu à l'état de sel d'addition d'acide, ou, dans le cas où l'on veut préparer un composé de formule I pour lequel $R^1$ représente un radical $-COR^2$, on acyle le composé Ia, ou un de sel d'addition d'acide de celui-ci, au moyen d'un agent d'acylation introduisant le radical $-COR^2$ et on isole le composé ainsi formé, ou on le transforme éventuellement en un sel d'addition d'acide acceptable du point de vue pharmacologique et on isole celui-ci.

**2.** Procédé selon la revendication 1, caractérisé en ce que la cyclisation est effectuée dans un solvant, dispersant ou diluant, à des températures comprises entre -10 et 40°C, de préférence entre 0 et 20°C, au moyen d'un agent de cyclisation qui, en solution aqueuse, donne au pH une valeur inférieure à 3.

**3.** Procédé selon les revendications 1 et/ou 2, caractérisé en ce qu'on acyle au moyen d'un agent d'acylation introduisant le radical $-CO-R^2$ dont le symbole $R^2$ représente un alkyle en $C_1$-$C_4$, un alcoxy en $C_1$-$C_4$, un phényle ou un phényle monosubstitué qui porte un atome de chlore ou un radical méthyle ou méthoxy.

**4.** Procédé selon les revendications 1 et/ou 2, caractérisé en ce que les composés de départ sont choisis de telle façon qu'il se forme un sel d'addition d'acide acceptable du point de vue pharmacologique, plus particulièrement le chlorhydrate de la 3-(3,3-diméthyltétrahydro-1,4-thiazine-4-yl)-sydnonimine.

**5.** Procédé selon les revendications 1 et/ou 2, caractérisé en ce que les composés de départ sont choisis de telle façon qu'il se forme la N-p-anisoyl-3-(3,3-diméthyl-tétrahydro-1,4-thiazine-4-yl)-sydnonimine ou l'un de ses sels d'addition d'acides acceptables du point de vue pharmacologique, plus particulièrement son chlorhydrate.

**6.** Procédé selon les revendications 1 et/ou 2, caractérisé en ce que les composés de départ sont choisis de telle façon qu'il se forme un sel d'addition d'acide acceptable du point de vue pharmacologique, plus particulièrement le chlorhydrate, de la 3-(3,3-diméthyltétrahydro-1,4-thiazine-1,1-dioxyde-4-yl)-sydnonimine.

**7.** Procédé pour préparer des 3-amino-sydnonimines substituées qui répondent à la formule générale Ib :

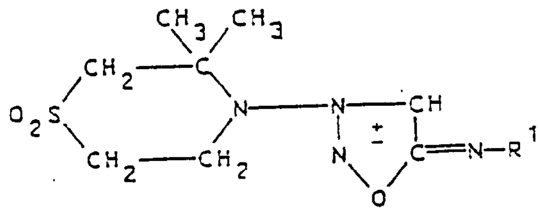

(Ib)

dans laquelle $R^1$ représente l'hydrogène ou un radical $-COR^2$, et $R^2$ représente un alkyle en $C_1$-$C_4$, un ($C_1$-$C_4$)alcoxy-($C_1$-$C_4$)alkyle, un alcoxy en $C_1$-$C_4$, un cycloalkyle en $C_5$-$C_7$, un phényle ou un phényle mono-, di- ou tri-substitué qui porte de 1 à 3 atomes d'halogènes et/ou de 1 à 3 radicaux alkyles contenant chacun de 1 à 4 atomes de carbone et/ou de 1 à 3 radicaux alcoxy contenant chacun de 1 à 4 atomes de carbone, et leurs sels d'addition d'acides acceptables du point de vue pharmacologique, procédé caractérisé en ce qu'on fait réagir un composé répondant à la formule générale Ic :

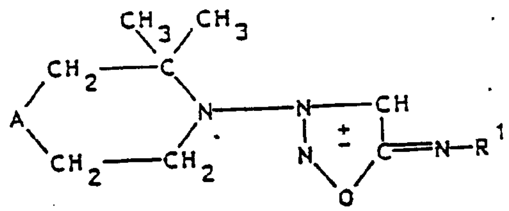

(Ic)

dans laquelle A représente un radical -S- ou -SO- et $R^1$ a la signification qui lui a été donnée ci-dessus, ou un sel d'addition d'acide d'un tel composé, avec un composé cédant de l'oxygène, on transforme éventuellement le composé ainsi obtenu en un sel d'addition d'acide et on l'isole, ou, dans le cas où l'on a obtenu un composé de formule I pour lequel A représente $-SO_2-$ et $R^1$ représente l'hydrogène et qu'on veut préparer un composé de formule I pour lequel A représente $-SO_2-$ et $R^1$ un radical $-COR^2$, on acyle le composé obtenu, ou un sel d'addition d'acide d'un tel composé, au moyen d'un agent d'acylation introduisant le radical $-COR^2$ et on isole le composé ainsi obtenu, ou on le transforme éventuellement en un sel d'addition d'acide acceptable du point de vue pharmacologique et on isole celui-ci.

8. Procédé selon la revendication 7, caractérisé en ce que les composés de départ sont choisis de telle façon qu'il se forme un sel d'addition d'acide acceptable du point de vue pharmacologique, plus particulièrement le chlorhydrate, de la 3-(3,3-diméthyl-tétrahydro-1, 4-thiazine-1,1-dioxyde-4-yl)-sydnonimine.

9. Application de 3-amino-sydnonimines substituées répondant à la formule générale I :

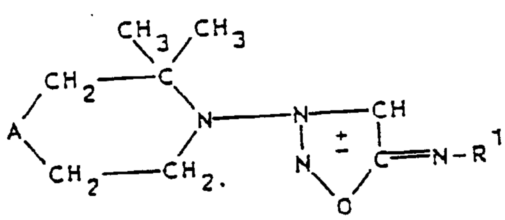

(I)

dans laquelle :

A représente un radical -S- ou $-SO_2-$,

$R^1$ représente un radical $-CO-R^2$ ou l'hydrogène, et

$R^2$ représente un alkyle en $C_1$-$C_4$, un ($C_1$-$C_4$) alcoxy-($C_1$-$C_4$) alkyle, un alcoxy en $C_1$-$C_4$, un cycloalkyle en $C_5$-$C_7$, un phényle ou un phényle monosubstitué, disubstitué ou trisubstitué qui porte de 1 à 3 atomes d'halogènes et/ou de 1 à 3 radicaux alkyles contenant de 1 à 4 atomes de carbone et/ou de 1 à 3 radicaux alcoxy contenant de 1 à 4 atomes de carbone,

et de leurs sels d'addition, acceptables du point de vue pharmacologique, qu'elles forment avec des acides, comme substances actives pharmacologiques pour la préparation de compositions pharmaceutiques.

10. Procédé pour préparer une composition pharmaceutique, procédé caractérisé en ce qu'on transforme de manière connue une ou plusieurs 3-amino-sydnonimines substituées répondant à formule générale I :

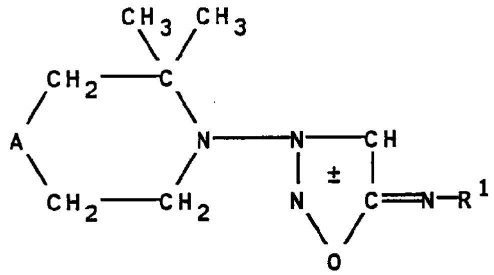

(I)

dans laquelle A représente un radical -S- ou $-SO_2-$, $R^1$ représente un radical $-CO-R^2$ ou l'hydrogène, et $R^2$ représente un alkyle en $C_1$-$C_4$, un ($C_1$-$C_4$)alcoxy($C_1$-$C_4$)alkyle, un alcoxy en $C_1$-$C_4$, un cycloalkyle en $C_5$-$C_7$, un phényle ou un phényle monosubstitué, disubstitué ou trisubstitué qui porte de 1 à 3 atomes d'halogènes et /ou de 1 à 3 radicaux alkyles contenant de 1 à 4 atomes de carbone et/ou 1 à 3 radicaux alcoxy contenant de 1 à 4 atomes de carbone,
et/ou un ou plusieurs de leurs sels d'addition d'acides acceptables de point de vue pharmacologique, comme substance(s) active(s), avec un ou plusieurs excipients et/ou additifs acceptables du point de vue pharmaceutique et, éventuellement, une ou plusieurs autres substances douées d'une activité pharmacologique, en une composition pharmaceutique convenant pour l'administration.